# FASCICULE DE BREVET EUROPEEN

(11) **EP 0 915 715 B1**
(45) Date de publication et mention de la délivrance du brevet: **10.09.2008**
(21) Numéro de dépôt: 97930561.2
(22) Date de dépôt: 20.06.1997
(51) Int. Cl.: A61M 3/02, A61B 17/32, B67D 5/02, B05B 9/047

(54) **Générateur-distributeur bi-fonctionde liquide notamment stérile**
Bifunktioneller Energieerzeuger und -verteiler von Flüssigkeiten, insbesondere von sterilen Flüssigkeiten
Bifunctional liquid dispensing generator, in particular for sterilised liquids

(30) Priorité: 21.06.1996 FR 9607907
(43) Date de publication de la demande: 19.05.1999
(73) Titulaire: Eschmann Holdings Limited, Lancing, West Sussex BN15 8TJ (GB)
(72) Inventeur: GONON, Bertrand, F-69002 Lyon (FR); SEZEUR, Alain, F-94230 Cachan (FR)
(74) Mandataire: Metz, Paul
(86) Numéro de dépôt international: PCT/FR1997/001108
(87) Numéro de publication internationale: WO 1997/049441

(56) Documents cités:
- WO-A-94/27659
- WO-A-94/28807
- DE-A- 3 726 788
- FR-A- 2 454 308
- GB-A- 646 167
- US-A- 2 669 233
- US-A- 3 963 024
- US-A- 4 457 487
- US-A- 4 662 871
- US-A- 4 913 698
- PATENT ABSTRACTS OF JAPAN vol. 013, no. 105 (C-575), 13 mars 1989 & JP 63 279832 A (SUGINO MACH:KK), 16 novembre 1988,

## Description

Le générateur-distributeur assure la délivrance d'un liquide notamment stérile à pression constante réglable ou à débit constant réglable.

Il délivre dans sa version adaptée un liquide stérile dans les conditions stériles telles qu'exigées par les applications médicales.

Le générateur est du type contenant souple placé dans une enceinte dans laquelle règne une pression déterminée d'un fluide gazeux inerte, de préférence non miscible au liquide contenu dans le contenant souple. Le gaz sous pression expulse le liquide à travers une pièce de raccordement et un conduit aboutissant à une pièce à main.

Pour diverses applications telles que d'une part par exemple l'aquadissection, l'injection de médicaments ou de produits de contraste et d'autre part le nettoyage ou le lessivage en profondeur, la désincrustation et autres, il s'avère nécessaire d'opérer à haute pression.

Le liquide devant sortir à haute pression à l'extrémité de la pièce à main nécessite d'abord une pression suffisante du gaz de compression, mais également un conduit de liaison du type haute pression avec la pièce à main et un orifice calibré disposé en sortie, de forme et de diamètre correspondant à la forme du jet approprié à l'application envisagée.

On connaît par la publication WO 94/28807 au nom de SAPHIR MEDICAL un appareil chirurgical d'aquadissection formé d'un générateur de sérum physiologique contenu dans une poche mise sous pression dans une enceinte étanche au moyen d'un gaz sous pression et raccordée à une pièce à main par un conduit souple, lui-même raccordé à une branche sortant de la poche par un percuteur stérile et traversant une des parois de l'enceinte par un raccord conique d'étanchéité.

Un circuit annexe aboutissant à la pièce à main est relié à une pompe aspirante en vue d'aspirer le liquide et d'évacuer les agrégats de tissus produits par l'opération d'aquadissection.

L'objectif de cette invention est unique. Il s'agit de procurer un générateur permettant d'effectuer des dissections chirurgicales à partir d'un jet de liquide stérile.

Pour arriver à ce but, le sérum physiologique doit être projeté à haute pression réglable manuellement par l'opérateur.

Les moyens employés sont tels que la pression à la sortie de la pièce à main ou pression de coupe reste constante. Cette exigence est réalisée par une carte électronique de régulation destinée à maintenir la pression constante dans l'enceinte étanche. La conséquence est le maintien constant de la pression de coupe à la valeur optimale pour le travail du chirurgien.

On peut citer également la publication EP 0 411 170 ou US 4,913,698 ITO qui concerne un générateur de ce type. On vise la production d'un jet concentré à haute pression pour permettre d'effectuer un travail d'aquadissection.

Il existe par ailleurs d'autres applications dans les domaines médicaux, chirurgicaux et domaines analogues ou autres pour lesquelles la haute pression est déconseillée. Il en est ainsi des pulvérisations, des rinçages, des aspersions, des humidifications, des traitements de désinfection par contact, des imprégnations, des lavages par ruissellement...

Ces autres applications nécessitent l'utilisation d'un générateur différent car le travail demandé au liquide n'est pas celui procuré par un effet de pression. Au contraire, on recherche un effet de débit sous faible pression.

Une illustration d'un générateur de débit correspond à la publication WO 94/27659 au nom de GUIGNARD.

Le générateur de liquide physiologique se compose également d'une poche contenant le liquide, poche soumise à une pression motrice dans une enceinte étanche. Cette pression provient d'une bouteille d'un gaz neutre sous pression. La pression de ce gaz est fortement limitée par un manodétendeur et régulateur. Une valve de sécurité est réglée à 30 kPa, soit 0,3 bar. Cette faible pression correspond à l'application endoscopique envisagée. En effet, on ne peut injecter dans le corps humain que des liquides sous faible pression.

Par contre, ce générateur délivre un débit important car il s'agit d'apporter un supplément conséquent de liquide de clarification pour améliorer la vision de la zone opératoire. Bien entendu, le liquide est extrait pour éviter le gonflement anormal de l'organe et la diffusion du liquide dans le corps d'où la présence d'une pompe péristaltique d'extraction.

Ce générateur est limité en pression et ne peut donc être utilisé pour des opérations où la pression est nécessaire au travail, par exemple la dissection.

En raison de la nature différente des besoins indiqués ci-dessus, ceux-ci ne peuvent être satisfaits par un seul générateur-distributeur raccordé par le même conduit à une pièce à main interchangeable en fonction de l'usage ou de l'application.

La présente invention a pour but, avec le même générateur-distributeur de liquide, de réaliser alternativement les deux fonctions ou les deux modes sans nécessiter de manipulations laborieuses difficiles et lentes mais avec une grande précision et dans des conditions de stérilité compatibles avec des applications médicales ou chirurgicales.

Plus particulièrement, la présente invention a pour objectif de réaliser un générateur-distributeur stérile bi-fonction et multiusage pouvant fonctionner en générateur de pression puis en générateur de débit par une simple modification.

L'invention a également pour but de fournir un générateur stérile bi-fonction dont la fonction en générateur de pression ou de débit est déterminée à la mise en service ou en cours de fonctionnement par le choix du tronçon de raccordement, de préférence jetable, ou par une commande sur un composant hydraulique, garantissant ainsi la stérilité et le mode bi-fonction à un coût largement inférieur à celui d'un générateur à deux fonctions juxtaposées.

A cet effet, l'invention se rapporte à un générateur-distributeur, tel que décrit dans la revendication 1*.*

Les avantages de ce générateur-distributeur sont multiples, non pas seulement dans les domaines médicaux et chirurgicaux, mais aussi dans des domaines très variés dans lesquels les exigences de stérilité sont plus ou moins rigoureuses, par exemple dans le domaine alimentaire.

Ainsi, moyennant le simple changement du tronçon de raccordement préférentiellement à usage unique et l'interposition d'un composant hydraulique, éventuellement déjà incorporé dans ce tronçon ou dans la pièce de jonction, ou la présence d'une conformation hydraulique, on transforme le générateur de pression en générateur de débit ou inversement, en vue d'une utilisation pour une autre application.

Tous les avantages classiques d'un appareil bi-fonction ou à fonction supplémentaire intégrée sont inhérents à l'invention. De plus le passage d'une fonction à une autre relève d'une grande simplicité.

En plus de celles déjà indiquées ci-dessus, différentes applications spécifiques dans les domaines médicaux et chirurgicaux sont données ci-après à titre d'exemple.

En application générateur de pression :
- pressions faibles et moyennes : (de 0 à 5 bars) : injection de médicaments liquides, pulvérisation pour lavage de plaies...
- pressions plus élevées (supérieures à 5 bars) : aquadissection, injection par cathéter de médicaments ou de produits de contraste, désincrustation, plans de clivage chirurgicaux.

En application générateur de débit à valeurs réglables entre 0 à 3 L/mn :
- lavages chirurgicaux et péritonéaux,
- irrigation en endoscopie.

Dans l'application particulière de la chirurgie, notamment digestive, le générateur selon l'invention permet d'une part de réaliser des dissections par jet de sérum physiologique à haute pression en chirurgie ouverte et en laparoscopie et d'autre part, sans changement de générateur, de réaliser des lavages péritonéaux en laparoscopie.

La pression ou le débit sont établis à une valeur constante mais réglable qui est celle demandée par le bouton de commande. Cette valeur peut être modifiée en cours de manipulation ou d'opération.

D'autres caractéristiques et avantages de l'invention apparaîtront dans la description qui suit, donnée à titre d'exemple et accompagnée des dessins dans lesquels :

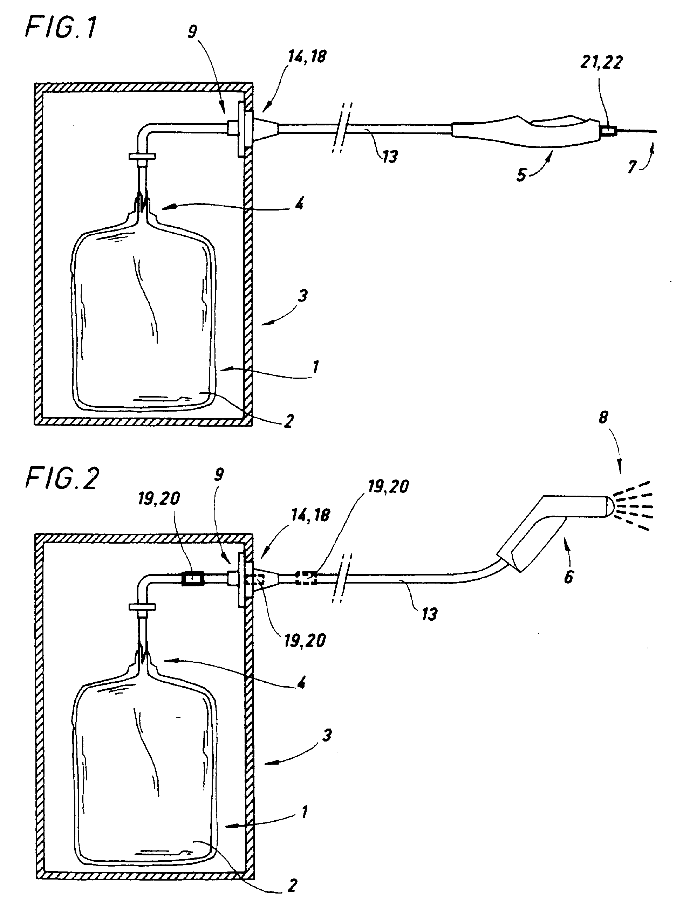

la figure 1 est une vue schématique d'ensemble en plan montrant le générateur et son contenant souple de liquide à distribuer selon un exemple de montage en générateur de pression ;
la figure 2 est une vue schématique d'ensemble en plan montrant le générateur et son contenant souple de liquide à distribuer selon un exemple de montage en générateur de débit sensiblement constant sur laquelle ont été représentés trois emplacements possibles pour le composant ou la conformation hydraulique ;

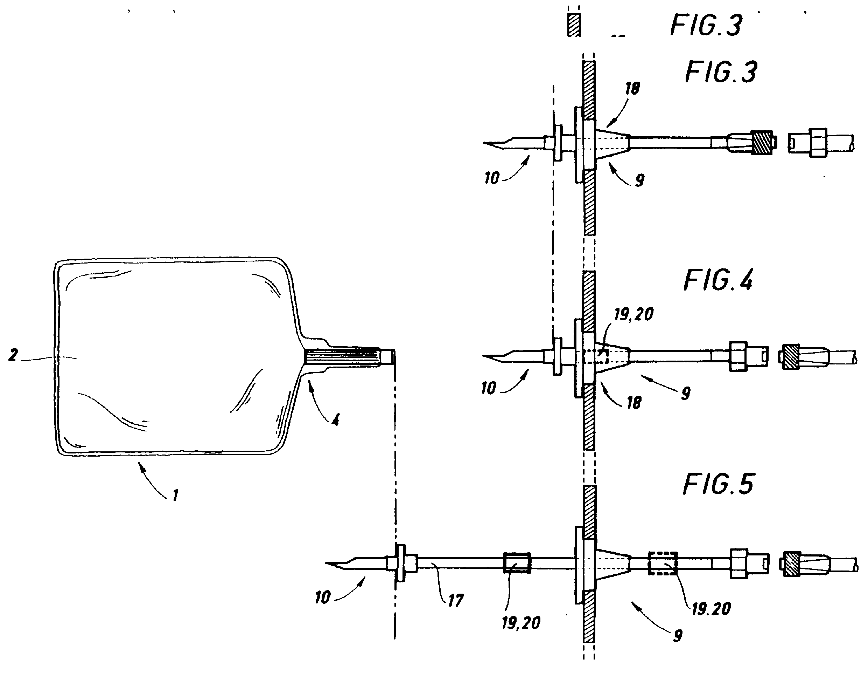

les figures 3, 4 et 5 sont des vues en plan montrant trois exemples de liaison dont deux avec cône-percuteur monobloc et liaison souple extérieure et un avec tronçon de raccordement respectivement dans le cas d'un générateur de pression (figure 3) et d'un générateur de débit (figures 4 et 5) ;

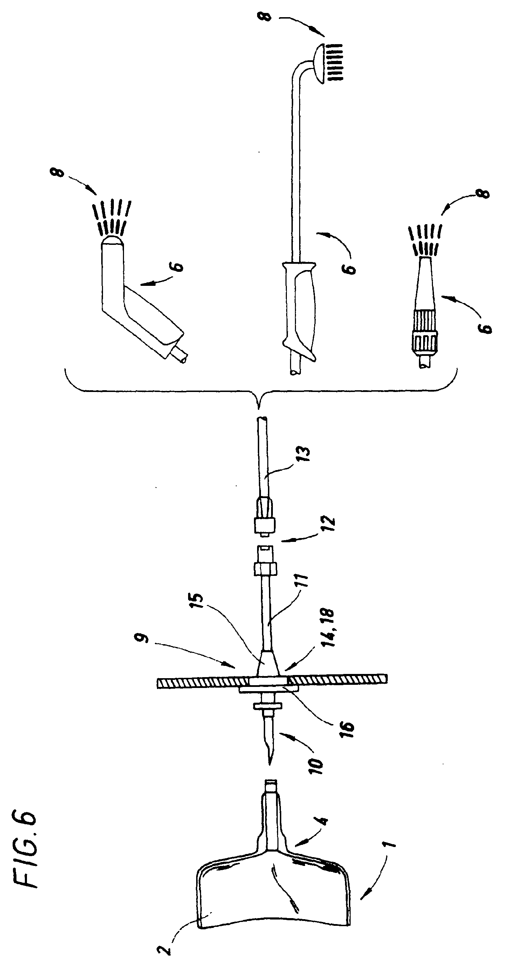

la figure 6 est une vue en plan illustrant une application en générateur de débit avec quelques exemples de pièces à main ;

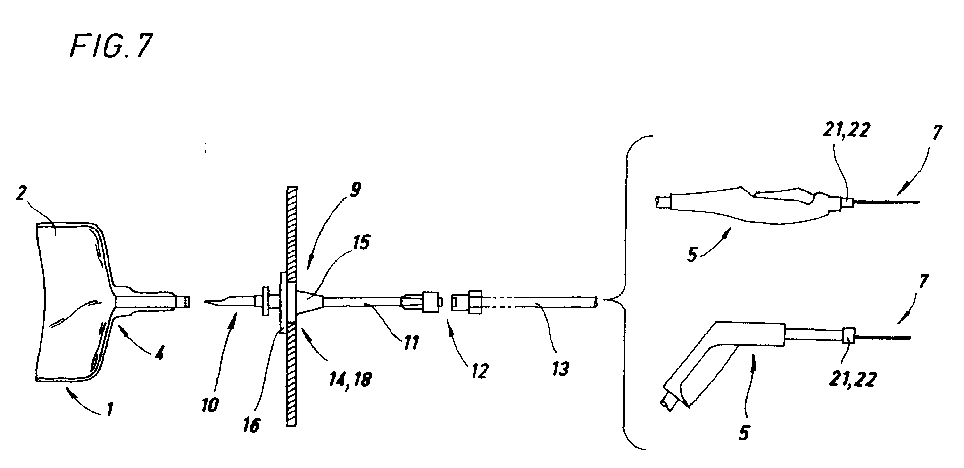

• la figure 7 est une vue en plan illustrant une application en générateur de pression avec quelques exemples de pièces à main ;
• la figure 8 est une vue schématique en coupe d'une pièce hydraulique à diaphragme incorporé commandé à distance ;
• la figure 9 est une vue schématique en coupe d'une pièce hydraulique à deux voies dont une au moins comporte une obturation commandée à distance ;
• la figure 10 est une vue schématique d'un tronçon hydraulique à partie souple étranglée par un collier ;
. la figure 11 est une vue schématique en perspective d'un tronçon hydraulique à partie étranglée par une pièce à ouverture double ;
. la figure 12 est une vue schématique en coupe d'une pièce d'étranglement du type cavalier ;
. la figure 13 est une vue schématique en coupe d'un étranglement par galet sur rampe oblique ;
. la figure 14 est une vue schématique en coupe d'un étranglement par deux doigts de pincement pivotants.

La présente invention , telle que décrite dans la revendication 1, concerne un générateur-distributeur de préférence stérile délivrant un liquide également de préférence stérile contenu dans une poche souple placée dans une enceinte soumise à la pression d'un gaz moteur. Cette poche souple est reliée à une pièce à main par les éléments suivants : un tronçon hydraulique de raccordement formé d'un percuteur suivi d'une pièce de traversée de l'enceinte et d'une liaison extérieure à l'enceinte aboutissant à un raccord avec un conduit souple d'alimentation de la pièce à main.

Dans sa version stérile l'ensemble des pièces constituant le générateur et le générateur lui-même sont stériles ou stérilisées.

Cette invention procède de l'idée générale inventive selon laquelle le tronçon hydraulique de raccordement reliant le contenant souple au raccord extérieur est remplacé partiellement ou totalement par une autre liaison, de préférence stérile et jetable, à chaque fois que l'on veut transformer le générateur de pression en un générateur de débit et inversement, ou le tronçon hydraulique de raccordement comprend au moins un composant hydraulique permettant de passer d'une fonction à une autre.

Un générateur-distributeur de liquide fonctionnant en générateur de pression ou en générateur de débit est formé à partir d'un contenant souple 1, par exemple d'une poche de préférence stérile remplie d'un liquide quelconque 2 de préférence stérile. La poche est enfermée dans une enceinte 3 étanche mise sous la pression d'un gaz moteur, de préférence inerte et non miscible avec le liquide à distribuer, par exemple de l'azote.

Les moyens extérieurs à l'enceinte étanche pour sa mise en pression sont supposés connus.

Cette poche stérile 1, remplie par exemple d'un liquide stérile par exemple du sérum physiologique dans le cas d'une application médicale ou chirurgicale, présente une sortie étroite en goulot 4, terminée par un embout obturé en extrémité par un opercule qui sera perforé lors de l'utilisation.

Le générateur-distributeur selon l'invention est destiné à délivrer alternativement, par une simple modification ou une commande, un liquide, sous pression sensiblement constante mais réglable ou à débit sensiblement constant mais réglable à une pièce à main à effet de pression 5 ou de débit 6 de forme et de fonction essentiellement variable dépendant de l'application.

La pièce à main de pression 5 délivre un jet compact 7 sous pression permettant d'utiliser l'effet de pression pour le travail, alors que la pièce à main de débit 6 délivre un jet diffus 8 pour les autres usages réclamant une plus grande quantité de liquide.

Selon l'application, mais surtout dans les domaines médicaux et chirurgicaux, le liquide ou soluté peut être chauffé jusqu'à une température limite qui sera maintenue et régulée afin d'éviter tout refroidissement par exemple d'une cavité à explorer.

La liaison hydraulique de la poche 1 avec l'extérieur s'effectue par un tronçon hydraulique de raccordement 9 terminé d'un côté par un percuteur 10 à extrémité en biseau destinée à perforer l'opercule et de l'autre côté après un conduit souple de sortie 11, par un raccord hydraulique 12 avec une liaison d'alimentation 13 jusqu'à la pièce à main 5,6 après avoir traversé la paroi adjacente de l'enceinte par une pièce traversante 14 du genre passe-fil logée dans une ouverture de la paroi. Cette pièce traversante assure simultanément l'étanchéité par rapport au gaz intérieur sous pression et par rapport à l'extérieur. La pièce traversante 14 présente par exemple un passage central traversé par le conduit du tronçon de raccordement 9.

Comme indiqué, ce tronçon de raccordement 9 présente une partie extérieure flexible 11 de faible longueur jusqu'au raccord hydraulique 12 du type deux pièces, destiné à le raccorder hydrauliquement, de préférence à diamètre constant, avec la liaison hydraulique d'alimentation 13 de la pièce à main 5,6.

Cette pièce traversante 14 est conformée par exemple en une demi-partie conique 15 suivie d'une embase 16 en forme de disque venant se plaquer en applique contre la paroi intérieure de l'enceinte tandis que l'extrémité conique de diamètre supérieur à l'ouverture dans la paroi de l'enceinte assure l'étanchéité par contact étroit de pression par rapport aux bords de l'ouverture et par enserrement autour du conduit de sortie.

Le tronçon de raccordement 9 peut également présenter une liaison intérieure 17 entre le percuteur 10 et la pièce traversante 14.

Dans la réalisation préférée représentée sur la majorité des figures, la partie interne du tronçon de raccordement 9 entre la paroi et la poche souple 1 contenant le liquide 2 est réalisée de façon compacte. Cette partie interne est en réalité d'une seule pièce et sera appelée ci-après cône-percuteur 18 pour cette variante préférée. Cette pièce unique peut remplir toutes les fonctions techniques du tronçon de raccordement 9.

Dans le cas d'un générateur de débit, un composant hydraulique 19 et/ou une conformation hydraulique 20, par exemple un simple passage du type orifice calibré, est inséré ou conformé en un endroit quelconque du tronçon de raccordement.

Dans le cas d'un générateur de pression, un composant hydraulique 21 et/ou une conformation hydraulique du type restriction 22, par exemple un simple passage du type orifice calibré de diamètre plus faible que celui de la liaison d'alimentation, est inséré ou conformé immédiatement à la sortie de la pièce à main 5 à effet de pression alors que le conduit hydraulique ne présente pas de restriction de liaison.

Ce composant hydraulique ou cette conformation est intégré(e) à, ou conformé(e) dans la pièce unique cône-percuteur 18 dans le cas de la variante avec cette pièce unique. Il peut s'agir d'un passage de diamètre précis (orifice calibré) dépendant de l'application et de la gamme de débits sensiblement constants que l'on veut obtenir.

Pour obtenir une gamme étendue de débits, le diamètre de cet orifice calibré sera sensiblement égal au diamètre du ou des conduits d'alimentation. On utilise alors une pièce à main 6 prévue pour fonctionner en débit, c'est-à-dire une pièce à main dont l'orifice de sortie ne comporte aucune restriction.

Pour obtenir une gamme inférieure ou plus restreinte de débits, on utilisera un orifice calibré de plus petit diamètre.

Cet orifice, lorsqu'il est intégré à, ou conformé dans la pièce unique, est choisi au moment du montage de mise en service.

Selon un avantage particulier de l'invention, lors d'une utilisation en générateur de pression, le passage calibré prévu pour un débit sensiblement constant peut rester dans le seul cas où son diamètre est suffisant, c'est-à-dire se rapproche de celui des conduits de liaison. Par contre, la pièce à main doit présenter à son extrémité une conformation en restriction 22, par exemple un orifice calibré de section notablement plus faible que celle des conduits de liaison.

La forme de section de cet orifice calibré dépend de l'application envisagée. Il peut s'agir d'un orifice circulaire destiné à créer un jet en aiguille pour des applications de chirurgie fine ou d'une forme en fente pour la formation d'une nappe pour des applications de nettoyage sous pression.

Selon le type d'application, le tronçon de raccordement 9 et/ou la liaison souple d'alimentation souple 13, et/ou le raccord 12 sont ou est à usage unique pour des raisons de stérilité liées aux applications médicales et alimentaires.

Bien entendu, il est envisageable que ces tronçons de raccordement 9 et les liaisons d'alimentation 13 puissent être réutilisés.

Pour éviter les erreurs de branchement déterminant la fonction en service, on peut prévoir des raccords 12 de formes différentes. Ces formes seront représentatives de l'une ou l'autre fonction de pression ou de débit. On peut prévoir par exemple un raccord 12 mâle côté enceinte dans le cas d'un générateur de pression et l'inverse dans le cas d'un générateur de débit comme représenté sur les figures de 3 à 5.

On peut prévoir également pour la fonction débit et pour la fonction pression au moins un composant hydraulique neutralisable ou commandé d'une position opérante à une position inopérante ou commandé progressivement vers des ouvertures de plus en plus grandes ou de plus en plus petites.

Par exemple le composant hydraulique limitant le débit, lorsqu'il constitue une restriction, peut devenir entièrement passant, c'est-à-dire à pleine ouverture sur simple commande. Le générateur-distributeur de liquide peut ainsi se transformer en générateur de pression sur simple commande extérieure.

Pour en obtenir les pleins effets, il suffit de raccorder le conduit souple de sortie à une pièce à main spécifique qui nécessite, pour fonctionner ou pour assurer pleinement sa fonction, une pression de liquide suffisante.

Il s'agit en particulier et à titre illustratif d'un instrument d'aquadissection comme représenté sur les figures.

On comprendra tout l'intérêt de l'invention qui consiste à passer avec le même appareil de base d'un générateur de débit à un générateur de pression et inversement, et ceci dans des conditions de stérilité compatibles avec les applications médicales.

On peut également, selon une variante élaborée de l'invention, passer en cours de fonctionnement d'une fonction à une autre.

Une autre variante, non représentée, consiste à remplacer le composant hydraulique de la partie flexible extérieure par une vanne trois voies ou à prévoir en plus à cet emplacement une vanne trois voies.

Le générateur-distributeur doit présenter en amont une ou des conformation(s) ou composant(s) hydraulique(s) assurant le fonctionnement de débit dans des conditions qui ne sont pas incompatibles avec la fonction de générateur de pression.

Le composant à trois voies peut également renfermer dans une même pièce l'orifice calibré de débit éventuellement réglable.

Selon cette variante, il suffit de raccorder à une des sorties de la vanne trois voies une pièce à main de pression 5 et à son autre sortie une pièce à main de débit 6.

L'appareil selon l'invention trouve ses applications préférentielles dans le domaine médical, par exemple d'une part en aquadissection (générateur de pression) et d'autre part en injection à débit constant dans le corps humain, par exemple de produits de contraste avant radiographie, angiographie, scintigraphie...

Bien entendu, de nombreuses autres applications peuvent être envisagées dans les domaines les plus variés, mais surtout dans les domaines où des conditions stériles sont exigées.

En effet, il suffit de remplacer le tronçon stérile de raccordement par un autre tronçon stérile adapté à la nouvelle application.

Le générateur est du type à poche souple comprimée par un gaz sous pression inerte non miscible avec le liquide que l'on distribue.

On peut envisager selon l'application d'autres gaz sous pression et même de l'air, de préférence stérile.

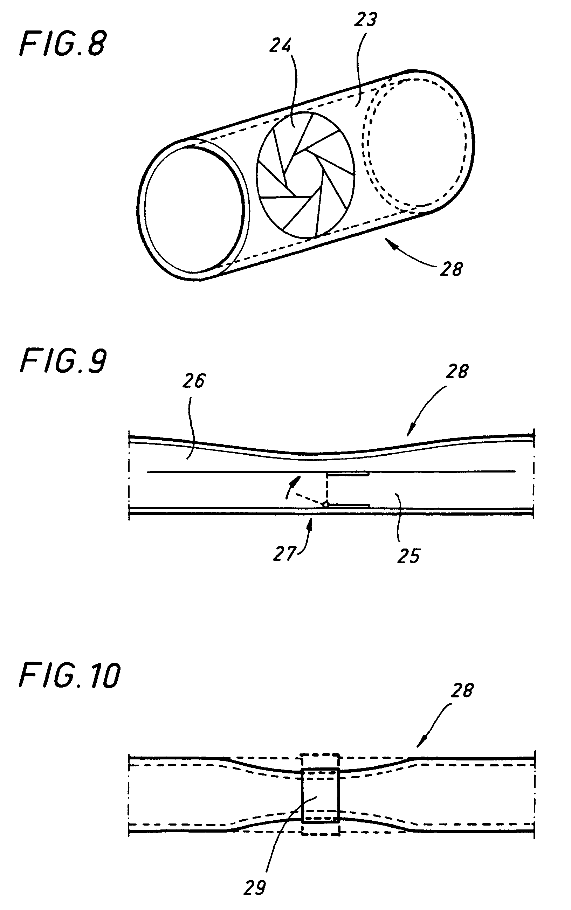

On peut également envisager comme représenté sur les figures 8 et 9 d'utiliser des pièces de raccordements bi-fonctionnelles, ou plus généralement des pièces hydrauliques bi-fonctionnelles, c'est-à-dire celles comportant une fonction commandée par exemple la restriction ou encore plus généralement des dispositifs de restriction imposant lorsqu'ils sont opérants une réduction de diamètre.

Sur la variante de la figure 8, il s'agit d'une pièce de raccordement 23 à diaphragme 24 intégré et commandé à distance. On peut réduire le diamètre de passage progressivement ou brutalement d'une valeur d'ouverture totale à une valeur réduite représentant la restriction calibrée imposée par le mode de fonctionnement en débit.

Il peut s'agir aussi, comme représenté sur la figure 9, d'une réalisation à deux voies dont l'une 25 à grand diamètre et l'autre 26 à diamètre réduit avec une obturation commandée à distance d'au moins une des voies par exemple par un clapet 27.

Une obturation sur la voie 25 de grand diamètre est nécessaire car la voie 26 à diamètre réduit placée en parallèle sur l'autre peut rester libre dans la fonction générateur de débit de pression.

Le tronçon 9 peut être constitué ou comporter une partie souple ou un tube souple 28 en matière souple, par exemple élastique susceptible d'étranglement par compression transversale ou annulaire sous la forme d'un pincement, d'un écrasement ou autre en vue de la réduction de diamètre du passage du liquide.

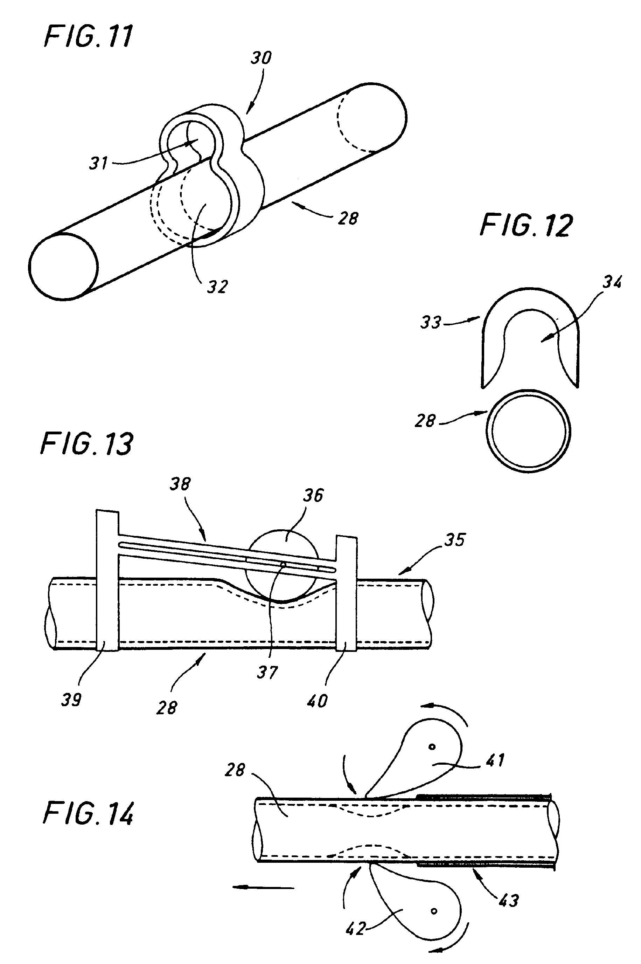

Le resserrement annulaire peut provenir d'un collier ou d'une pièce annulaire déformable 29 (figure 10). Il peut provenir aussi d'une pièce d'étranglement 30 comportant en succession latérale deux ouvertures successives 31 et 32 dont l'une 31 de diamètre égal à celui de la partie souple ou du tube souple 28 du tronçon et dont l'autre 32 de plus petit diamètre (figure 11). Cette pièce est mobile transversalement par rapport à la partie souple ou au tube souple 28 du tronçon.

Cette pièce d'étranglement à double ouverture 31 et 32 peut être remplacée par une pièce d'étranglement en étrier 33 de plus faible passage intérieur 34 que le diamètre de la partie souple 28 du tronçon (figure 12). Cet étrier 33 est mobile transversalement à la partie souple 28 du tronçon.

La restriction par étranglement obtenue par resserrement, pincement ou écrasement peut provenir également de différents dispositifs d'étranglement dont les deux dispositifs suivants représentés sur les figures 13 et 14 à titre d'exemple.

Il s'agit d'abord du dispositif d'étranglement 35 à galet 36 représenté sur la figure 13. Ce dispositif 35 permet un écrasement local de la partie souple 28 du tronçon par le rapprochement mutuel entre la surface latérale du galet 36 et le tube ou partie souple 28. Ce rapprochement provient du déplacement de l'axe 37 du galet 36 sur une rampe oblique descendante 38. Cette rampe oblique est soutenue par deux pièces transversales d'extrémité 39 et 40 comme représenté sur la figure 13.

Il s'agit ensuite, comme représenté sur la figure 14, de deux doigts pivotants de pincement 41 et 42 dont les extrémités sont en contact avec la surface latérale de la partie souple 28 du tronçon, partie suivie d'une zone rigide 43. Ces doigts pivotants 41 et 42 sont montés chacun pivotant en rappel élastique l'un vers l'autre contre le tube selon une position rapprochée de pincement par exemple à travers un cliquet. Une traction de déplacement sur le tube dans le sens de la flèche le fera échapper au pincement en translatant la zone rigide 43 entre les doigts de pincement 41 et 42 qui ne peuvent la déformer. Ce dispositif peut être prévu pour assurer un pincement prédéterminé correspondant à la restriction nécessaire pour le passage dans l'autre fonction.

## Revendications

1. Générateur-distributeur de liquide, notamment stérile, alimentant une pièce à mais (5,6) pour des applications chirurgicales ou médicales respectivement à haute pression ou en débit, le générateur-distributeur formé à partir d'un contenant souple (1) fermé ou autoalimenté par une réserve contenant le liquide (2) et enfermé dans une enceinte (3) sous pression d'un gaz, ce contenant (1) étant relié à l'extérieur par un tronçon de raccordement (9) qui présente à l'une de ses extrémités un percuteur (10) destiné à crever la paroi latérale de la poche du contenant souple (1) et à venir s'adapter à l'extrémité de celle-ci, le tronçon de raccordement (9) traversant une paroi de l'enceinte (3) par une pièce traversante (14) étanche au gaz sous pression régnant dans l'enceinte (3) et étant relié par son autre extrémité à une pièce à main (5,6) qui délivre le liquide respectivement en haute pression ou en débit, **caractérisé en ce que** le générateur-distributeur de liquide permet avec le même appareil de réaliser alternativement une fonction de haute pression ou de débit, **en ce que** pour cela le tronçon de raccordement (9) est :
- soit un tronçon de raccordement (9) comportant au moins un moyen (19,20) remplissant la fonction de limiteur de pression transformant par sa présence le générateur-distributeur en générateur de débit pour la pièce à main (6),
- soit un tronçon de raccordement (9) ne comportant pas de moyen remplissant la fonction de limiteur de pression transformant le générateur-distributeur en générateur de pression pour la pièce à main (5),
et **en ce que** le tronçon de raccordement (9) permettant la réalisation de l'une des fonctions est amovible et remplaçable par le tronçon de raccordement (9) permettant la réalisation de l'autre fonction.

2. Générateur-distributeur selon la revendication 1 **caractérisé en ce que** le tronçon de raccordement (9) est à usage unique.

3. Générateur-distributeur selon la revendication précédente **caractérisé en ce que** le tronçon de raccordement (9) est stérile.

4. Générateur-distributeur selon la revendication 1 **caractérisé en ce que** le tronçon de raccordement (9) comprend un composant hydraulique limiteur de pression.

5. Générateur-distributeur selon la revendication 1 **caractérisé en ce que** le tronçon de raccordement (9) comprend une conformation hydraulique intérieure au conduit destinée à limiter la pression du liquide stérile dans le tronçon de raccordement (9).

6. Générateur-distributeur selon la revendication 4 ou 5 **caractérisé en ce que** le tronçon de raccordement (9) comprend un composant hydraulique ou une conformation hydraulique limitant la pression à un emplacement proche de la paroi de l'enceinte.

7. Générateur-distributeur selon l'une quelconque des revendications précédentes de 4 à 6 **caractérisé en ce que** le composant hydraulique ou la conformation hydraulique est un orifice calibré circulaire.

8. Générateur-distributeur selon l'une quelconque des revendications précédentes **caractérisé en ce que** le composant hydraulique ou la conformation hydraulique est une restriction de la section de passage du conduit située immédiatement à l'extrémité du tronçon de raccordement du côté de la pièce à main.

9. Générateur-distributeur selon la revendication précédente **caractérisé en ce que** la restriction est un orifice calibré circulaire ou une fente.

10. Générateur-distributeur l'une des revendications précédentes 1 à 4 **caractérisé en ce que** le composant hydraulique est un composant comportant un orifice calibré situé sur le tronçon de raccordement (9).

11. Générateur-distributeur selon l'une des revendications précédentes **caractérisé en ce que** le percuteur (10) et la pièce traversante (14) ne forment qu'une seule pièce dite cône-percuteur (18).

12. Générateur-distributeur selon la revendication précédente **caractérisé en ce que** le composant hydraulique est dans le cône-percuteur (18) ou celui-ci présente une conformation hydraulique.

13. Générateur-distributeur selon l'une quelconque des revendications précédentes **caractérisé en ce que** le composant hydraulique du tronçon (9) est une vanne à trois voies.

14. Générateur-distributeur selon la revendication précédente **caractérisé en ce que** la vanne à trois voies comporte un composant hydraulique ou présente une conformation hydraulique imposant des conditions de débit.

15. Générateur-distributeur selon l'une quelconque des revendications précédentes **caractérisé en ce que** le raccord de connexion entre le tronçon de raccordement (9) et la liaison d'alimentation (13) de la pièce à main est d'un type pour la fonction de générateur de pression et d'un autre type pour la fonction de générateur de débit.

16. Générateur-distributeur selon la revendication 1 **caractérisé en ce que** le tronçon de raccordement (9) comporte deux voies (25) et (26), l'une à diamètre normal et l'autre à diamètre réduit, une des deux voies comportant un obturateur (27) commandé à distance.

17. Générateur-distributeur selon la revendication précédente **caractérisé en ce que** la voie à diamètre normal comporte seule un obturateur (27) commandé à distance.

18. Générateur-distributeur selon la revendication 1 **caractérisé en ce que** le tronçon de raccordement (9) comporte une partie (28) en matière souple se déformant par resserrement radial pour la réduction du diamètre ou est en tube souple (28).

19. Générateur-distributeur selon la revendication précédente **caractérisé en ce que** la réduction de diamètre de passage est provoquée par une pièce d'étranglement.

20. Générateur-distributeur selon la revendication précédente **caractérisé en ce que** la réduction de diamètre de passage provoquée par étranglement provient du resserrement d'une pièce annulaire déformable (29) montée sur le tube souple ou la partie souple (28) du tronçon.

21. Générateur-distributeur selon la revendication précédente **caractérisé en ce que** la réduction du diamètre provient d'une pièce à ouverture.

22. Générateur-distributeur selon la revendication précédente **caractérisé en ce que** la pièce à ouverture est une pièce (30) à double ouverture, une grande (32) et une petite (31), décalées latéralement qui vient s'adapter sur la partie souple (28) du tronçon et est mobile transversalement par rapport à ce tronçon.

23. Générateur-distributeur selon la revendication précédente **caractérisé en ce que** la pièce à ouverture est un étrier (33) dont l'écartement des branches délimite un passage étroit (34) utilisé pour réaliser un pincement de restriction.

24. Générateur-distributeur selon la revendication 19 **caractérisé en ce que** la réduction de diamètre provoquée par étranglement provient de la pression de la surface latérale d'un galet (36) sur le tube souple ou la partie souple (28) du tronçon monté à rotation sur une rampe oblique (38).

25. Générateur-distributeur selon la revendication 19 **caractérisé en ce que** la réduction de diamètre provoquée par étranglement provient du pincement de deux doigts pivotants (41) et (42) montés pivotants en rappel élastique de pincement l'un vers l'autre de part et d'autre d'une partie souple (28) du tronçon suivie par une zone rigide (43) qui vient se déplacer entre les doigts lors d'une traction sur le tronçon permettant le passage dans l'autre fonction.

## Claims

1. A generator-distributor for liquid, particularly for sterile liquid, supplying a hand piece (5, 6), for respectively high pressure or flow delivery surgical or medical applications, the generator-distributor using a closed or self-fed from a reserve flexible container (1) containing the liquid (2) and being enclosed in a gas pressurized chamber (3), this container (1) being connected to the exterior with a connecting section (9) with a pin (10) at one end, which punctures the lateral wall of the flexible container (1) pouch and which adapts to its extremity, said connecting section (9) passing through one wall of the chamber (3) by a seal (14) tight to the pressurized gas of the chamber (3) and being connected with its other end to a hand piece (5) which delivers the liquid respectively under high pressure or flow delivery, **characterized in that** the liquid generator-distributor allows with the same apparatus to realize alternatively a high pressure function or a flow delivery function; **in that** the connecting section (9) is for that reason:
- either a connecting section (9) comprising at least one pressure limiting means (19, 20) transforming by its presence the generator-distributor into a flow delivery generator for the hand piece (6),
- or a connecting section (9) comprising no pressure limiting means (19, 20) transforming the generator-distributor into a high pressure generator for the hand piece (5);
and **in that** the connecting section (9) allowing one of the functions to take place is detachable and may be replaced by a connection section (9) allowing the other function to take place.

2. A generator-distributor according to claim 1, **characterized in that** the connecting section (9) is a single-use piece.

3. A generator-distributor according to the preceding claim, **characterized in that** the connecting section (9) is sterile.

4. A generator-distributor according to claim 1, **characterized in that** the connecting section (9) comprises a pressure limiting hydraulic component.

5. A generator-distributor according to claim 1, **characterized in that** the connecting section (9) comprises a hydraulic conformation in the conduit which limits the pressure of the sterile liquid in the connecting section (9).

6. A generator-distributor according to claim 4 or 5, **characterized in that** the connecting section (9) comprises a pressure limiting hydraulic component or hydraulic conformation positioned near the wall of the chamber.

7. A generator-distributor according to any one of preceding claims 4 to 6, **characterized in that** the hydraulic component or the hydraulic conformation is a calibrated circular orifice.

8. A generator-distributor according to any one of the preceding claims, **characterized in that** the hydraulic component or the hydraulic conformation is a constriction in the passage section of the conduit, situated immediately at the end of the connecting section towards the hand piece.

9. A generator-distributor according to the preceding claim, **characterized in that** the constriction is a calibrated circular orifice or a slit.

10. A generator-distributor according to any one of preceding claims 1 to 4, **characterized in that** the hydraulic component is a component comprising a calibrated orifice located on the connecting section.

11. A generator-distributor according to any one of the preceding claims, **characterized in that** the pin (10) and the seal (14) form a single element called the cone-pin (18).

12. A generator-distributor according to the preceding claim, **characterized in that** the hydraulic component is in the cone-pin (18) or the cone-pin has a hydraulic conformation.

13. A generator-distributor according to any one of the preceding claims, **characterized in that** the hydraulic component of the connecting section (9) is a triple track valve.

14. A generator-distributor according to the preceding claim, **characterized in that** the triple track valve comprises a hydraulic component or has a hydraulic conformation determining flow conditions.

15. A generator-distributor according to any one of the preceding claims, **characterized in that** the connector between the connecting section (9) and the supply inlet (13) of the hand piece is a particular type for the pressure generating function and a different type for the flow delivery generating function.

16. A generator-distributor according to claim 1, **characterized in that** the connecting section (9) has two tracks (25) and (26), one of normal diameter and one of reduced diameter, one of these tracks comprising a block (27) which is remotely controlled.

17. A generator-distributor according to the preceding claim, **characterized in that** only the normal diameter track comprises a block (27) which is remotely controlled.

18. A generator-distributor according to claim 1, **characterized in that** the connecting section (9) comprises a portion (28) made of flexible material which can be deformed by radial constriction to reduce its diameter, or this portion consists of a flexible tube (28).

19. A generator-distributor according to the preceding claim, **characterized in that** the passage diameter reduction is caused by a constricting element.

20. A generator-distributor according to the preceding claim, **characterized in that** the passage diameter reduction caused by constriction results from constriction of a deformable annular piece (29) attached to the flexible tube or the flexible portion (28) of the section.

21. A generator-distributor according to the preceding claim, **characterized in that** the diameter reduction results from a piece with opening.

22. A generator-distributor according to the preceding claim, **characterized in that** the piece with opening is a piece (30) with double openings, one large (32) and one small (31), laterally offset, adapted to the flexible portion (28) of the section and transversely movable with respect to said section.

23. A generator-distributor according to the preceding claim, **characterized in that** the piece with opening is a bracket (33) with branch spacing delimiting a narrow passageway (34) used to realize a restriction pinching.

24. A generator-distributor according to claim 19, **characterized in that** the diameter reduction caused by constriction results from pressure of the lateral surface of a roller (36) on the flexible tube or the flexible portion (28) of the section, said roller rotating along an oblique plane (38).

25. A generator-distributor according to claim 19, **characterized in that** the diameter reduction caused by constriction results from compression by two pivoting fingers (41) and (42) which are pivotably attached and can be elastically forced together towards each other on both sides of a flexible portion (28) of the section followed by a rigid area (43) which moves to a position between the fingers when the section is pulled to allow the other function to occur.

## Patentansprüche

1. Gerät zum Erzeugen und Verteilen einer Flüssigkeit, insbesondere einer sterilen Flüssigkeit, die ein Handgerät (5, 6) für chirurgische oder medizinische Anwendungen jeweils mit Hochdruck oder Durchflussregelung versorgt, wobei der Erzeuger-Spender aus einem biegsamen Behälter (1) gebildet ist, der verschlossen ist oder mit einer die Flüssigkeit (2) enthaltenden Reserve automatisch eingespeist wird und in einer unter Gasdruck stehenden Einfassung (3) eingeschlossen ist, wobei dieser Behälter (1) außenseitig durch einen Verbindungsabschnitt (9) verbunden ist, der an einem seiner Enden einen Schlagbolzen (10) aufweist, der dazu bestimmt ist, die Seitenwand der biegsamen Behältertasche (1) aufzustechen und sich an das Ende der letzteren anzupassen, wobei der Verbindungsabschnitt (9) mit einem Durchführungsstück (14), das gegen das in der Einfassung (3) herrschenden Druckgases abgedichtet ist, durch eine Wand der Einfassung (3) hindurchführt und über sein anderes Ende mit einem Handgerät (5, 6) verbunden ist, das die Flüssigkeit jeweils unter Hochdruck oder in der Durchflussmenge auslässt, **gekennzeichnet dadurch, dass** der Erzeuger-Spender der Flüssigkeit es gestattet, mit demselben Gerät alternativ eine Hochdruck- oder Durchflussregelungs-Funktion zu vollbringen, **und dadurch, dass** hierfür der Verbindungsabschnitt (9)
- entweder ein Verbindungsabschnitt (9) ist mit mindestens einem Mittel (19, 20), das die Funktion als Druckbegrenzungsventil ausübt, wobei sich der Erzeuger-Spender durch seine Anwesenheit in einen Durchflussgenerator für das Handgerät (6) umwandelt,
- oder ein Verbindungsabschnitt (9) ist, der kein Mittel umfasst, das die Funktion als Druckbegrenzungsventil ausübt, wobei sich der Erzeuger-Spender in einen Druckgenerator für das Handgerät (5) umwandelt,
**und dadurch, dass** der Verbindungsabschnitt (9), der die Vollstreckung der Funktionen ermöglicht, abnehmbar und durch den Verbindungsabschnitt (9) austauschbar ist, der es ermöglicht, die andere Funktion durchzuführen.

2. Erzeuger-Spender nach Anspruch 1, **gekennzeichnet dadurch, dass** der Verbindungsabschnitt (9) für einen einzigen Gebrauch bestimmt ist.

3. Erzeuger-Spender nach dem vorherigen Anspruch, **gekennzeichnet dadurch, dass** der Verbindungsabschnitt (9) steril ist.

4. Erzeuger-Spender nach Anspruch 1 **gekennzeichnet dadurch, dass** der Verbindungsabschnitt (9) eine hydraulische Komponente, nämlich ein Druckbegrenzungsventil, umfasst.

5. Erzeuger-Spender nach Anspruch 1 **gekennzeichnet dadurch, dass** der Verbindungsabschnitt (9) eine innere hydraulische Anordnung an der Leitung umfasst, die dazu bestimmt ist, den Druck der sterilen Flüssigkeit in dem Verbindungsabschnitt (9) zu begrenzen.

6. Erzeuger-Spender nach Anspruch 4 oder 5 **gekennzeichnet dadurch, dass** der Verbindungsabschnitt (9) eine Hydraulik-Komponente oder eine hydraulische Anordnung umfasst und den Druck an einer der Wand der Einfassung naheliegenden Stelle begrenzt.

7. Erzeuger-Spender nach einem der vorherigen Ansprüche 4 bis 6, **gekennzeichnet dadurch, dass** die hydraulische Komponente oder die hydraulische Anordnung eine rund kalibrierte Öffnung ist.

8. Erzeuger-Spender nach einem der vorherigen Ansprüche, **gekennzeichnet dadurch, dass** die hydraulische Komponente oder die hydraulische Anordnung eine Einengung des Übergangsbereichs der Leitung ist, die direkt am Ende des handgerätseitigen Verbindungsabschnitts liegt.

9. Erzeuger-Spender nach dem vorherigen Anspruch, **gekennzeichnet dadurch, dass** die Einengung eine rund kalibrierte Öffnung oder ein Spalt ist.

10. Erzeuger-Spender gemäß einem der vorherigen Ansprüche 1 bis 4 **gekennzeichnet dadurch, dass** die hydraulische Komponente eine Komponente mit einer kalibrierten Öffnung ist, die sich auf dem Verbindungsabschnitt (9) befindet.

11. Erzeuger-Spender nach einem der vorherigen Ansprüche, **gekennzeichnet dadurch, dass** der Schlagbolzen (10) und das Durchführungsteil (14) nur ein einziges Teil, den sogenannten Zapfen-Schlagbolzen (18), bildet.

12. Erzeuger-Spender nach dem vorherigen Anspruch, **gekennzeichnet dadurch, dass** die hydraulische Komponente im Zapfen-Schlagbolzen (18) liegt oder diese eine hydraulische Anordnung aufweist.

13. Erzeuger-Spender nach einem der vorherigen Ansprüche, **gekennzeichnet dadurch, dass** die hydraulische Komponente des Abschnitts (9) ein Dreiwege-Ventil ist.

14. Erzeuger-Spender nach dem vorherigen Anspruch, **gekennzeichnet dadurch, dass** das Dreiwege-Ventil eine hydraulische Komponente umfasst oder eine hydraulische Anordnung aufweist, die die Durchflussbedingungen vorgibt.

15. Erzeuger-Spender nach einem der vorherigen Ansprüche, **gekennzeichnet dadurch, dass** der Verbindungsanschluss zwischen dem Verbindungsabschnitt (9) und der Einspeisungsverbindung (13) des Handgeräts von einem Typ ist, der als Druckgenerator fungiert und von einem anderen Typ ist, der als Durchflussgenerator fungiert.

16. Erzeuger-Spender nach Anspruch 1, **gekennzeichnet dadurch, dass** der Verbindungsabschnitt (9) zwei Wege (25) und (26) umfasst, und zwar der eine mit normalem Durchmesser und der andere mit verringertem Durchmesser, wobei einer der beiden Wege mit einem fernbedienten Absperrorgan (27) versehen ist.

17. Erzeuger-Spender nach dem vorherigen Anspruch, **gekennzeichnet dadurch, dass** der Weg mit normalem Durchmesser nur ein einziges fernbedientes Absperrorgan (27) umfasst.

18. Erzeuger-Spender nach Anspruch 1, **gekennzeichnet dadurch, dass** der Verbindungsabschnitt (9) einen Teil (28) aus biegsamem Material umfasst, der sich durch radiale Verengung verformt zur Reduzierung des Durchmessers oder er besteht aus einem flexiblen Rohr (28).

19. Erzeuger-Spender nach dem vorherigen Anspruch, **gekennzeichnet dadurch, dass** die Reduzierung des Durchgangs-Durchmessers durch ein Drosselorgan bewirkt wird.

20. Erzeuger-Spender nach dem vorherigen Anspruch, **gekennzeichnet dadurch, dass** die durch eine Drosselung bewirkte Reduzierung des Durchgangs-Durchmessers von der Verengung eines verformbaren ringförmigen Teils (29) herrührt, das auf das flexible Rohr oder auf das biegsame Teil (28) des Abschnitts montiert ist.

21. Erzeuger-Spender nach dem vorherigen Anspruch, **gekennzeichnet dadurch, dass** die Reduzierung des Durchmessers von einem Öffnungsteil herrührt.

22. Erzeuger-Spender nach dem vorherigen Anspruch, **gekennzeichnet dadurch, dass** das Öffnungsteil ein Teil (30) mit doppelter Öffnung ist, und zwar einer seitlich versetzten großen (32) und kleinen Öffnung (31), die an das biegsame Teil (28) des Abschnitts angepasst ist und quer zu diesem Abschnitt hin beweglich ist.

23. Erzeuger-Spender nach dem vorherigen Anspruch, **gekennzeichnet dadurch, dass** das Öffnungsteil ein Verbindungsstück (33) ist, dessen Abstand von den Abzweigkanälen einen engen Durchgang (34) begrenzt, der benutzt wird, um eine Abklemmung vorzunehmen.

24. Erzeuger-Spender nach Anspruch 19, **gekennzeichnet dadurch, dass** die durch Drosselung bewirkte Reduzierung des Durchmessers auf den Druck der Seitenfläche einer Laufrolle (36) auf das flexible Rohr oder das flexible Teil (28) des drehbar auf einer schrägen Auflauffläche (38) montierten Abschnitts zurückführt.

25. Erzeuger-Spender nach Anspruch 19, **gekennzeichnet dadurch, dass** die durch Drosselung bewirkte Reduzierung des Durchmessers durch Einklemmen von zwei drehbaren Fingern (41) und (42) zustande kam, die drehbar elastisch klemmend federnd einer in Richtung zum anderen beiderseits eines flexiblen Teils (28) des Abschnitts angebracht sind, dem ein starrer Bereich (43) folgt, der sich zwischen den Fingern aufgrund einer Zugkraft auf dem Abschnitt fortbewegt und so die Passage in der anderen Funktion gestattet.
